# EUROPEAN PATENT APPLICATION

(11) **EP 4 492 398 A1**
(43) Date of publication of application: **15.01.2025**
(21) Application number: 23767095.5
(22) Date of filing: 06.03.2023
(51) Int. Cl.: G16H 50/20, G16H 10/60, G16H 10/40, G16H 50/30, G16H 20/30, G16H 20/70, A61B 5/12, G06N 3/02, A61M 21/00, A61F 11/00

(54) **DIAGNOSTIC METHOD AND CLASSIFICATION METHOD FOR PATIENTS WITH TINNITUS, AND METHOD FOR PROVIDING PERSONALIZED TREATMENT PROTOCOLS FOR PATIENTS WITH TINNITUS**

(30) Priority: 07.03.2022 KR 20220028674
(71) Applicant: Neurive Co., Ltd., Juchon-myeon Gimhae-si, Gyeongsangnam-do 50969 (KR)
(72) Inventor: SONG, Jae Jun, Seoul 06501 (KR); HONG, Ki Hwan, Seoul 03709 (KR)
(74) Representative: Franke, Dirk
(86) International application number: PCT/KR2023/003000
(87) International publication number: WO 2023/171994

(57) **Abstract**

A diagnostic method and a classification method for patients with tinnitus, and a method for providing personalized treatment protocols for the patients with the tinnitus are proposed. The diagnostic method for the tinnitus patients includes an information input step for inputting information including patient information, tinnitus test results, and medical interview results, and a diagnosis step for deriving a scale for tinnitus symptoms, a scale for tinnitus-related negative thoughts, and a scale for tinnitus-related negative emotions from the information, and diagnosing each patient on the basis of the scale for the tinnitus symptoms, the scale for the tinnitus-related negative thoughts, and the scale for the tinnitus-related negative emotions, wherein a medical interview questionnaire includes question items about the tinnitus-related negative thoughts and question items about the tinnitus-related negative emotions, and the diagnostic step is performed by using a deep learning-based artificial intelligence algorithm.

## Description

### Technical Field

The present disclosure relates to a method of providing personalized treatment protocols for patients and, more particularly, to a method of diagnosing or classifying tinnitus patients and providing personalized treatment protocols suitable for each patient.

### Background Art

In general, tinnitus refers to a state in which a person hears a certain sound in a situation where there is no external auditory stimulation. It has been known that tinnitus mainly occurs concurrently with hearing loss, and that the tinnitus occurs when the cerebrum compensates for the inability to receive normal sound stimulation due to the hearing loss. However, various studies have confirmed that there are more causes of tinnitus other than the hearing loss, and these causes may include stress, fatigue, noise, otitis externa, etc.

Since tinnitus has various causes and also diverse symptoms, treatment is conducted differently for each patient. In particular, many efforts have been made to conduct personalized treatments to patients according to their tinnitus symptoms (in Korean Patent No. 10-1057661).

However, there are many differences in treatment effectiveness depending on patients, and accordingly, various treatment methods have been developed. Currently, more than 15 treatment methods are being used in South Korea. For example, there are provided the treatment methods including drug therapy, tinnitus masking therapy, implantable hearing aid surgery, regular counseling therapy, etc.

Tinnitus patients account for approximately 10% of the total population (estimated at approximately five million people) and approximately 30% of the elderly population, but there is no established treatment yet, and tinnitus retraining treatment, which consists of sound therapy and counseling, is the most widely used clinically. However, currently, such various tinnitus treatment methods are not effectively personalized and provided to the patients. The reason is that although many studies have been conducted on personalized treatment based on tinnitus symptoms, selecting particular treatment methods based on causes such as psychological factors above the symptoms is just performed on the basis of a doctor's intuition without any special standards.

### Disclosure

### Technical Problem

The present disclosure is for solving the problems of the related art described above, and an objective of the present disclosure is to provide a method of determining optimal tinnitus treatment protocols by reflecting various factors affecting tinnitus treatment.

### Technical Solution

According to the present disclosure to achieve the above objective, there is provided a diagnostic method for tinnitus patients, the diagnostic method including: an information input step for inputting information including patient information, tinnitus test results, and medical interview results; and a diagnosis step for deriving a scale for tinnitus symptoms, a scale for tinnitus-related negative thoughts, and a scale for tinnitus-related negative emotions from the information, and diagnosing each patient on the basis of the scale for the tinnitus symptoms, the scale for the tinnitus-related negative thoughts, and the scale for the tinnitus-related negative emotions, wherein a medical interview questionnaire includes question items about the tinnitus-related negative thoughts and question items about the tinnitus-related negative emotions, and the diagnostic step is performed by using a deep learning-based artificial intelligence algorithm.

In this case, the scale for the tinnitus symptoms may be derived by reflecting the tinnitus test results.

The scale for the tinnitus-related negative thoughts and the scale for the tinnitus-related negative emotions may be derived by reflecting the medical interview results.

According to another aspect of the present disclosure, there is provided a classification method for tinnitus patients, the classification method including: an information input step for inputting information including patient information, tinnitus test results, and medical interview results; a diagnosis step for deriving a scale for tinnitus symptoms, a scale for tinnitus-related negative thoughts, and a scale for tinnitus-related negative emotions from the information, and diagnosing each patient on the basis of the scale for the tinnitus symptoms, the scale for the tinnitus-related negative thoughts, and the scale for the tinnitus-related negative emotions; and a classification step for classifying the patients into a plurality of treatment groups on the basis of diagnostic information about the patients, wherein a medical interview questionnaire includes question items about the tinnitus-related negative thoughts and question items about the tinnitus-related negative emotions, and the diagnostic step and the classification step are performed by using deep learning-based artificial intelligence algorithms.

In this case, the scale for the tinnitus symptoms may be derived by reflecting the tinnitus test results.

The scale for the tinnitus-related negative thoughts and the scale for the tinnitus-related negative emotions may be derived by reflecting the medical interview results.

The treatment groups may be composed of a functional treatment group, an emotional treatment group, and a cognitive treatment group.

According to a yet another aspect of the present disclosure, there is provided a method of providing personalized treatment protocols for tinnitus patients, the method including: an information input step for inputting information including patient information, tinnitus test results, and medical interview results; a diagnosis step for deriving a scale for tinnitus symptoms, a scale for tinnitus-related negative thoughts, and a scale for tinnitus-related negative emotions from the information, and diagnosing each patient on the basis of the scale for the tinnitus symptoms, the scale for the tinnitus-related negative thoughts, and the scale for the tinnitus-related negative emotions; and a treatment protocol provision step for providing the personalized treatment protocols for the patients on the basis of diagnosis results, wherein a medical interview questionnaire includes question items about the tinnitus-related negative thoughts and question items about the tinnitus-related negative emotions, each treatment protocol is a treatment process composed of a plurality of treatment methods, the treatment protocol provision step provides the personalized treatment protocols for the patients by combining the treatment methods selected from a functional treatment method group, an emotional treatment method group, and a cognitive treatment method group on the basis of the diagnosis results, and the diagnostic step and the treatment protocol provision step are performed by using deep learning-based artificial intelligence algorithms.

According to a still another aspect of the present disclosure, there is provided a method of providing personalized treatment protocols for tinnitus patients, the method including: an information input step for inputting information including patient information, tinnitus test results, and medical interview results; a diagnosis step for deriving a scale for tinnitus symptoms, a scale for tinnitus-related negative thoughts, and a scale for tinnitus-related negative emotions from the information, and diagnosing each patient on the basis of the scale for the tinnitus symptoms, the scale for the tinnitus-related negative thoughts, and the scale for the tinnitus-related negative emotions; a classification step for classifying the patients into a plurality of treatment groups on the basis of diagnostic information about the patients; and a treatment protocol provision step for providing the personalized treatment protocols for the patients on the basis of classification results, wherein a medical interview questionnaire includes question items about the tinnitus-related negative thoughts and question items about the tinnitus-related negative emotions, each treatment protocol is a treatment process composed of a plurality of treatment methods, the treatment protocol provision step provides the personalized treatment protocols for the patients by combining the treatment methods selected from a functional treatment method group, an emotional treatment method group, and a cognitive treatment method group on the basis of the classification results, and the diagnostic step, the classification step, and the treatment protocol provision step are performed by using deep learning-based artificial intelligence algorithms.

The treatment groups may be composed of a functional treatment group, an emotional treatment group, and a cognitive treatment group.

The scale for the tinnitus symptoms may be derived by reflecting the tinnitus test results.

The scale for the tinnitus-related negative thoughts and the scale for the tinnitus-related negative emotions may be derived by reflecting the medical interview results.

As items that alleviate or directly treat the tinnitus symptoms, the functional treatment method group may include sound therapy and tinnitus retraining therapy.

The emotional treatment method group may include meditation therapy, relaxation therapy, sleep hygiene therapy, mindfulness therapy, and acceptance and commitment therapy.

The cognitive treatment method group may include thought record therapy and educational therapy on cognitive factors.

### Advantageous Effects

In the present disclosure configured as described above, there are effects of performing diagnosis and classification by reflecting emotional and cognitive factors of patients through artificial intelligence algorithms, so that not only the patients may be accurately diagnosed and classified, but also the diagnosis and classification of the patients may be performed automatically, thereby diagnosing and classifying the patients more quickly.

In addition, unlike the process of reflecting emotional or cognitive factors that depend on a doctor's intuition in applying the conventional treatment methods to patients, there is an effect of improving treatment effectiveness by generating and providing treatment protocols through artificial intelligence algorithms.

### Description of Drawings

FIG. 1 is a flowchart illustrating a process of diagnosing and classifying patients in order to provide personalized treatment protocols in an exemplary embodiment of the present disclosure.
FIGS. 2 and 3 are flowcharts illustrating processes of providing personalized treatment protocols according to exemplary embodiments of the present disclosure.

### Mode for Invention

Exemplary embodiments according to the present disclosure will be described in detail with reference to the accompanying drawings.

However, the embodiment forms of the present disclosure may be modified in many different forms, and the scope of the present disclosure is not limited only to the embodiment forms described below. The shapes and sizes of components in the drawings may be exaggerated for clearer description, and components indicated by the same reference numerals in the drawings are the same components.

In addition, throughout the specification, when a part is said to be "connected" to another part, an expression such as "connected" is intended to include not only "directly connected" but also "electrically connected", while having a different component in the middle thereof. In addition, when a part is said to "include" or "comprise" a certain component, it means that it may further include or comprise another component, without excluding another component, unless the context clearly indicates otherwise.

In addition, terms such as "first", "second", and the like are used to distinguish one component from another, so the scope of rights should not be limited by these terms. For example, a first component may be referred to as a second component, and similarly, the second component may also be referred to as the first component.

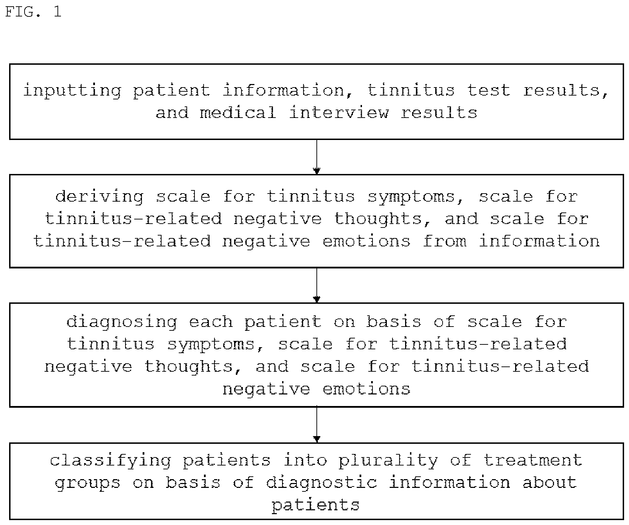

FIG. 1 is a flowchart illustrating a process of diagnosing and classifying patients in order to provide personalized treatment protocols in an exemplary embodiment of the present disclosure.

In the present disclosure, information is first input for diagnosis and classification of tinnitus patients.

The input information is personal information, tinnitus test results, and medical interview results of the patients.

The personal information of each patient includes the age and gender of each patient, and may include a history of accident or surgery, etc. of each patient.

Tinnitus measurement results are the results of testing each patient's tinnitus condition and include hearing threshold, tinnitus frequency, tinnitus loudness, and tinnitus disorder scale.

The medical interview results are the results of conducting a medical interview using a questionnaire related to tinnitus with patients, and the questionnaire includes question items about tinnitus-related negative thoughts and question items about tinnitus-related negative emotions.

The question items about tinnitus-related negative thoughts are question items about negative thoughts that arise due to tinnitus, and are prepared to receive answers from patients on content such as "I will get dementia because of tinnitus," "Tinnitus cannot be cured," and "Tinnitus means that there is a serious illness in my head."

The items for such negative thoughts may be composed so as to cause patients to answer the items in terms of number or frequency of how much they think about the corresponding content.

The question items about tinnitus-related negative emotions are prepared to receive answers from patients on the content such as "I feel anxious because of my tinnitus", "I feel depressed because of my tinnitus", and "I feel irritated because of my tinnitus".

The question items for such negative emotions may be composed so as to cause patients to answer the items in terms of number or frequency of how often they experience their feelings about the corresponding content.

Next, the patients are diagnosed and classified on the basis of the input information.

In the present disclosure, criteria for diagnosing and classifying patients are based on the features of the treatment methods, and to this end, the embodiments of the present disclosure classify various tinnitus treatment methods into a functional treatment method group, an emotional treatment method group, and a cognitive treatment method group.

As methods that alleviate tinnitus symptoms or directly treat tinnitus, the functional treatment method group includes sound therapy and auditory rehabilitation.

The sound therapy is a therapy for primarily using a method of playing natural sounds, music, environmental sounds, or the like to patients with tinnitus in order to treat suffering caused by the tinnitus.

The hearing rehabilitation is a therapy for reducing discomfort caused by tinnitus and hearing loss through methods such as wearing hearing aids, speech listening training, and improving of listening strategies in noisy environments.

As treatment methods for alleviating or resolving emotional problems caused by tinnitus, the emotional treatment method group includes meditation therapy, relaxation therapy, sleep hygiene therapy, mindfulness therapy, and acceptance and commitment therapy.

As a treatment method that reduces stress and promotes mental and physical stability by activating parasympathetic nerves through meditation, the Meditation therapy has an effect of reducing stress caused by tinnitus.

As a treatment method that performs training for reducing stress by reducing systemic muscle tension, the relaxation therapy has an effect of replenishing a human body's energy by conducting relaxation response training. In the human body, in addition to the central nervous system (i.e., the brain and spinal cord) and peripheral nervous system, there is an autonomic nervous system. The autonomic nervous system is divided into a sympathetic nervous system and a parasympathetic nervous system, which are always balanced through opposing responses. However, when people are stressed a lot or excessively concentrate, the sympathetic nervous system becomes dominant, resulting in "stress responses" such as a faster pulse, increase in blood pressure, increase in breathing rate, and increase in muscle tension. In contrast, when the relaxation response training is used, the parasympathetic nervous system becomes dominant, resulting in the opposing responses, namely "relaxation responses" such as a decrease in pulse, decrease in blood pressure, decrease in breathing rate, and decrease in muscle tension. The relaxation therapy has the following effects, ultimately improving the effectiveness of tinnitus treatment:
(1) Reducing fatigue and aiding ability to cope with worries
(2) Reducing stress that may cause high blood pressure, arteriosclerosis, heart attack, and stroke
③ Helping people quit smoking and drinking
④ Having ability to be used in relieving insomnia
⑤ Preventing waste of body energy
⑥ Making people more lucid and enhancing their concentration

As a treatment method that improves sleep, the sleep hygiene therapy has an effect of relieving symptoms that worsen due to the vicious cycle of insomnia and tinnitus in a case where people experience insomnia due to the tinnitus. The sleep hygiene therapy may be conducted by using a method of educating people on the following principles to improve sleep.

| |
|---|
| 1. Go to bed and wake up at the same time every day. |
| 2. Make sure your sleeping environment is quiet and not too bright. Avoid being too hot or too cold. |
| 3. Exercise regularly every day. However, exercising too much right before bed can make your sleep more difficult. |
| 4. Avoid drinks or foods containing caffeine. |
| 5. Avoid smoking or drinking alcohol before bed. Alcohol is thought to induce sleep at first, but it can cause frequent awakenings in the second half of sleep and can worsen sleep apnea syndrome. |
| 6. Taking a warm bath before bed can help. |
| 7. Avoid being hungry or overeating. |
| 8. Looking at the clock or looking at your mobile phone frequently in bed can interfere with sleep. |
| 9. Watching TV, reading, eating, or doing other things in bed can interfere with sleep. Try to do only sleeping in bed. |
| 10. If you can't fall asleep or wake up in the middle of the night and can't fall asleep, get out of bed and do something else. If you feel sleepy while doing something else, go to bed then. |
| 11 Avoid exposure to bright light at night. |

The mindfulness therapy is a treatment method that controls secondary responses to primary stimulation through mindfulness meditation. Usually, we first feel stimulation and then have secondary responses to the stimulation (i.e., primary stimulation → secondary responses). However, it may be said that the mindfulness meditation is a meditation that allows us to feel the primary stimulation (i.e., sensation, thought, etc.) as it is, but at the same time, allows us to try to avoid unnecessary the secondary responses against the primary stimulation (in a direction of solely performing necessary secondary responses). In the mindfulness meditation, it is known that distracting thoughts, suffering, etc. are concretized as specific sensations or thoughts (i.e., primary stimulation or primary sensation) originally occurred in the body or mind are arbitrarily responded (i.e., secondary responses) to the specific stimulation (i.e., the sensation and thoughts) by the human brain and body in a different form than originally intended (e.g., reflexes (i.e., conditioned reflexes and unconditioned reflexes), behaviorism, classical conditioning). In other words, it means that all things and the world itself are staying still, but the results of humans responding by themselves to all the things and the world are the distracting thoughts, the suffering, etc. Among the famous proverbs, "Mountains are mountains, rivers are rivers" comes from such a meaning. Therefore, the purpose of this meditation is to continuously observe all physical sensations and thoughts in the body and to not respond (i.e., non-response) to the stimulation of those corresponding sensations, thoughts, etc. as much as possible.

The acceptance and commitment therapy is a treatment method that allows therapeutic change to occur when a functional aspect, rather than a form, of personal experience is changed by changing primary context in which certain types of behavior (i.e., thoughts and emotions) influence other types of behavior (i.e., overt behavior). Through treatment strategies for accepting painful experiences rather than trying to suppress or eliminate the same and performing commitment behavior based on personal values, the acceptance and commitment therapy helps patients to have the workability of treatment throughout each patient's life.

As treatment items for alleviating or resolving cognitive problems caused by tinnitus, the cognitive treatment method group includes a thought record therapy and an educational therapy on cognitive factors.

The thought record therapy is a treatment method that records situations in which tinnitus-related negative emotions occur, records the negative thoughts that arise during these situations, and corrects the negative emotions and thoughts with positive thoughts.

The educational therapy for the cognitive factors is an educational counseling treatment method that corrects the content of negative thoughts about tinnitus into positive ones.

In the present disclosure, the reason that the features of treatment items are applied as criteria for diagnosing and classifying patients is to compensate for the fact that not only most tinnitus patients suffer from both emotional and cognitive problems caused by tinnitus symptoms, but also sufficient therapeutic effectiveness is not achieved due to the emotional and cognitive problems occurred when treatment is solely conducted simply on the tinnitus symptoms.

In the conventional method, functional treatment, emotional treatment, and cognitive treatment were selectively conducted depending on an intuition of a counselor or doctor through his or her experience, but the embodiment of present disclosure has applied the functional, emotional, and cognitive problems corresponding to the classification of treatment items as the criteria for the diagnosis and classification of patients in order to objectively assign appropriate treatment items to the patients. In addition, the process of diagnosing the patients on the basis of the input information may be performed with a deep learning-based artificial intelligence algorithm.

In the process of diagnosing patients by the artificial intelligence algorithm, the "scale for tinnitus symptoms (i.e., the functional scale)" may be derived from the tinnitus test results, and the "scale for tinnitus-related negative thoughts (i.e., the cognitive scale)" and "scale for the tinnitus-related negative emotions (i.e., the emotional scale)" may be derived from the medical interview results, whereby the scales may be reflected in the process. The "scale for tinnitus symptoms (i.e., the functional scale)" is the results of quantifying the tinnitus symptoms of the patients, and the "scale of tinnitus-related negative thoughts (the cognitive scale)" and the "scale for tinnitus-related negative emotions (the emotional scale)" are the results of quantifying the content of answers to the question items about the tinnitus-related negative thoughts and the question items about the tinnitus-related negative emotions among the question items of medical interview.

In a case where the answers to the question items about the tinnitus-related negative thoughts and the question items about the tinnitus-related negative emotions are received in numerical form, answer values may be used as it is or the answer values may be applied with a separate formula to derive the scales. In a case where the answers are not in the numerical form, the answers may be converted into the numeric form and then used.

The tinnitus test results for deriving the "scale for tinnitus symptoms (i.e., the functional scale)" may allow that tinnitus test methods developed to date and result data obtained therefrom are applied without limitation, and a technology to be developed thereafter may also be applied without limitation as long as it does not impair the features of the present disclosure. However, a process may be added to correct or standardize differences depending on expression methods of the result data.

A high value on the functional scale indicates a condition that a treatment corresponding to the functional treatment method group is mainly required, a high value on the cognitive scale indicates a condition that a treatment corresponding to the cognitive treatment method group is mainly required, and a high value on the emotional scale indicates a condition that a treatment corresponding to the emotional treatment method group is mainly required.

The artificial intelligence algorithm for diagnosing patients may be one that collects data on the results of tinnitus diagnosis previously performed on the patients as training data, and may be one that is trained through deep learning. In this case, the training data may be tinnitus diagnosis data reflecting the functional scale, cognitive scale, and emotional scale for each patient, and may also be one that includes treatment result data along with the tinnitus test results and medical interview results without content about each scale. In a case where there is no data for each scale, the artificial intelligence algorithm may assign a functional scale, a cognitive scale, and an emotional scale by learning which treatment is most effective for each patient through the treatment result data.

Through the above-described process, unlike the conventional method of diagnosing patients solely on the basis of tinnitus symptoms, the embodiments of the present disclosure are able to perform tinnitus patient diagnosis that reflects even psychological factors in addition to the tinnitus symptoms of the patients. Because of reflecting the diagnosis of the psychological factors, the diagnosis according to the present disclosure has a suitable form to provide, in the form optimized for each patient, treatment protocols including both the emotional treatment method group and cognitive treatment method group in addition to the functional treatment method group.

In this case, the treatment protocols may be generated on the basis of the diagnosis results for each patient as described above, but personalized treatment protocols may also be generated for each group after classifying and grouping the patients.

In order to group the patients, a process of classifying the patients may be added on the basis of the functional scale, cognitive scale, and emotional scale of each patient.

For example, patients may be classified into three treatment groups: a functional treatment group that requires functional treatment first, an emotional treatment group that requires emotional treatment first, and a cognitive treatment group that requires cognitive treatment first. Since this classification is performed by using the functional scale, cognitive scale, and emotional scale, which are diagnosed for each patient, it is also possible to classify the patients into a larger number of treatment groups than the above described treatment groups when required. In addition, a process of classifying the patients on the basis of the functional scale, cognitive scale, and emotional scale for each patient may be performed with a deep learning-based artificial intelligence algorithm.

The artificial intelligence algorithm for classifying patients may be one that collects, as training data, data on the results of tinnitus diagnosis and patient classification, which are previously performed on the patients, and may be one that is trained through deep learning. In this case, the training data may include both of tinnitus diagnosis data for each patient and result data of patient classification performed by a doctor, or may include tinnitus diagnosis data and treatment result data thereof. In a case of no classification result data, the artificial intelligence algorithm may classify the patients by learning which treatment is most effective for each patient through the treatment result data.

The artificial intelligence algorithm for diagnosing functional, cognitive, and emotional scales for patients and the artificial intelligence algorithm for classifying the patients may be trained in a mutually complementary manner. For example, in the process of performing learning that assigns a functional scale, a cognitive scale, and an emotional scale for each patient through treatment result data, first, the artificial intelligence algorithm may learn the process of classifying patients by learning which treatment is most effective for each patient through the treatment result data.

As described above, in the case where the deep learning-based artificial intelligence algorithms diagnose and classify patients on the basis of information composed of personal information of each patient, tinnitus test results, and medical interview results, not only the patients may be diagnosed and classified more accurately without misclassification, such as by missing some of the information, but also the diagnosis and classification of the patients are performed automatically, so the patients may be diagnosed and classified more quickly.

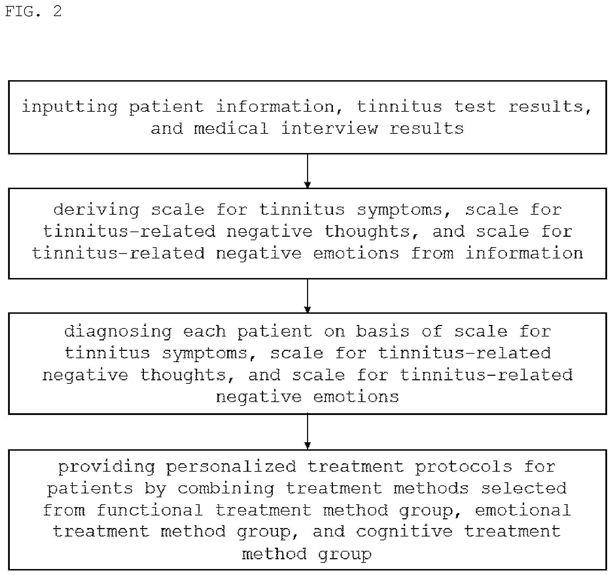

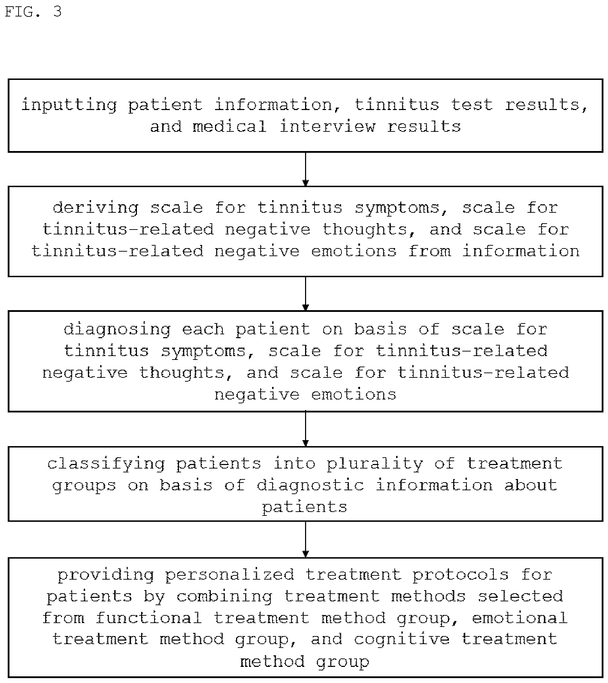

FIGS. 2 and 3 are flowcharts illustrating processes of providing personalized treatment protocols according to exemplary embodiments of the present disclosure.

Up to the steps of inputting information for diagnosis and classification related to tinnitus and diagnosing and classifying patients on the basis of the input information, the configuration thereof is the same as that of the diagnosis and classification methods described above, so detailed descriptions are omitted.

After the diagnosis and classification of the patients are performed, the treatment protocols are matched to the classified patients. Each treatment protocol is a treatment process composed of a plurality of treatment methods, and treatment items included in the treatment protocols are selected from the functional treatment method group, emotional treatment method group, and cognitive treatment method group.

In this case, the treatment items included in the treatment protocols vary depending on the diagnosis results of respective patients or the treatment groups to which respective patients belong.

For example, as described above, in the case of being classified into three treatment groups: the functional treatment group, the emotional treatment group, and the cognitive treatment group, the treatment items required for each treatment group are selected first, but are not limited thereto, and other treatment items may be included in the treatment protocols. In the case of the functional treatment group, the functional treatment method group is mainly included, but the emotional treatment method group or cognitive treatment method group may also be included. In the case of the emotional treatment group as well, the emotional treatment method group is mainly included, but the functional treatment method group or cognitive treatment method group may also be included. In the case of the cognitive therapy group as well, the cognitive treatment method group is mainly included, but the functional treatment method group or emotional treatment method group may also be included.

As described above, the process of generating the treatment protocols according to the classification of the patients may be performed with a deep learning-based artificial intelligence algorithm.

In addition, in a case of dividing treatment groups into three or more treatment groups, the treatment protocols may be generated by using various method such as a method of differently composing the number of items included in the three treatment items for each treatment group.

Furthermore, the treatment protocols matched to respective treatment groups are not fixed, and the treatment items included in each treatment protocol may be changed by way of reflecting treatment results. Specifically, in the deep learning process of the artificial intelligence algorithm that generates the treatment protocols according to the classification of the patients, a treatment protocol provided for each classification may also be changed by reflecting information on the treatment results.

In the case of generating the treatment protocols on the basis of the diagnosis result for each patient, treatments selected from among the functional treatment method group, cognitive treatment method group, and emotional treatment method group are composed by reflecting the functional scale, cognitive scale, and emotional scale for each patient. In this case, a weight may be assigned to each scale, and the treatment protocols may also be generated such that values for each scale are divided into a plurality of areas and treatment items are selected according to each area.

The treatment protocols provided to respective patients are not fixed, and the treatment items included in each treatment protocol may be changed by reflecting the treatment results. Specifically, in the deep learning process of the artificial intelligence algorithm that generates the treatment protocols for respective patients, the treatment protocols provided for respective patients may also be changed by reflecting information on the treatment results.

In addition to changing treatment protocols by reflecting the treatment results, related information may be used to reflect the diagnosis and classification of patients as well. Specifically, in the deep learning process of the artificial intelligence algorithms that perform the diagnosis and classification of the patients, the diagnosis and classification criteria may also be changed by reflecting the information on the treatment results.

According to the content described above, unlike that the process of reflecting emotional or cognitive factors is depended on a doctor's intuition in applying the conventional treatment methods to patients, there is an effect of improving treatment effectiveness by generating and providing the treatment protocols through the artificial intelligence algorithms.

The present disclosure has been described above through the preferred exemplary embodiments, but the above-described exemplary embodiments are only illustrative of the technical idea of the present disclosure, and those skilled in the art will appreciate that various changes are possible without departing from the scope of the technical idea of the present disclosure. Therefore, the protection scope of the present disclosure should be construed by the matters described in the claims, not by the specific examples, and all technical ideas within the scope equivalent thereto should be construed as being included in the scope of the present disclosure.

## Claims

1. A diagnostic method for tinnitus patients, the diagnostic method comprising:
an information input step for inputting information comprising patient information, tinnitus test results, and medical interview results; and
a diagnosis step for deriving a scale for tinnitus symptoms, a scale for tinnitus-related negative thoughts, and a scale for tinnitus-related negative emotions from the information, and diagnosing each patient on the basis of the scale for the tinnitus symptoms, the scale for the tinnitus-related negative thoughts, and the scale for the tinnitus-related negative emotions,
wherein a medical interview questionnaire comprises question items about the tinnitus-related negative thoughts and question items about the tinnitus-related negative emotions, and
the diagnostic step is performed by using a deep learning-based artificial intelligence algorithm.

2. The diagnostic method of claim 1, wherein the scale for the tinnitus symptoms is derived by reflecting the tinnitus test results.

3. The diagnostic method of claim 1, wherein the scale for the tinnitus-related negative thoughts and the scale for the tinnitus-related negative emotions are derived by reflecting the medical interview results.

4. A classification method for tinnitus patients, the classification method comprising:
an information input step for inputting information comprising patient information, tinnitus test results, and medical interview results;
a diagnosis step for deriving a scale for tinnitus symptoms, a scale for tinnitus-related negative thoughts, and a scale for tinnitus-related negative emotions from the information, and diagnosing each patient on the basis of the scale for the tinnitus symptoms, the scale for the tinnitus-related negative thoughts, and the scale for the tinnitus-related negative emotions; and
a classification step for classifying the patients into a plurality of treatment groups on the basis of diagnostic information about the patients,
wherein a medical interview questionnaire comprises question items about the tinnitus-related negative thoughts and question items about the tinnitus-related negative emotions, and
the diagnostic step and the classification step are performed by using deep learning-based artificial intelligence algorithms.

5. The classification method of claim 4, wherein the scale for the tinnitus symptoms is derived by reflecting the tinnitus test results.

6. The classification method of claim 4, wherein the scale for the tinnitus-related negative thoughts and the scale for the tinnitus-related negative emotions are derived by reflecting the medical interview results.

7. The classification method of claim 4, wherein the treatment groups are composed of a functional treatment group, an emotional treatment group, and a cognitive treatment group.

8. A method of providing personalized treatment protocols for tinnitus patients, the method comprising:
an information input step for inputting information comprising patient information, tinnitus test results, and medical interview results;
a diagnosis step for deriving a scale for tinnitus symptoms, a scale for tinnitus-related negative thoughts, and a scale for tinnitus-related negative emotions from the information, and diagnosing each patient on the basis of the scale for the tinnitus symptoms, the scale for the tinnitus-related negative thoughts, and the scale for the tinnitus-related negative emotions; and
a treatment protocol provision step for providing the personalized treatment protocols for the patients on the basis of diagnosis results,
wherein a medical interview questionnaire comprises question items about the tinnitus-related negative thoughts and question items about the tinnitus-related negative emotions,
each treatment protocol is a treatment process composed of a plurality of treatment methods,
the treatment protocol provision step provides the personalized treatment protocols for the patients by combining the treatment methods selected from a functional treatment method group, an emotional treatment method group, and a cognitive treatment method group on the basis of the diagnosis results, and
the diagnostic step and the treatment protocol provision step are performed by using deep learning-based artificial intelligence algorithms.

9. A method of providing personalized treatment protocols for tinnitus patients, the method comprising:
an information input step for inputting information comprising patient information, tinnitus test results, and medical interview results;
a diagnosis step for deriving a scale for tinnitus symptoms, a scale for tinnitus-related negative thoughts, and a scale for tinnitus-related negative emotions from the information, and diagnosing each patient on the basis of the scale for the tinnitus symptoms, the scale for the tinnitus-related negative thoughts, and the scale for the tinnitus-related negative emotions;
a classification step for classifying the patients into a plurality of treatment groups on the basis of diagnostic information about the patients; and
a treatment protocol provision step for providing the personalized treatment protocols for the patients on the basis of classification results,
wherein a medical interview questionnaire comprises question items about the tinnitus-related negative thoughts and question items about the tinnitus-related negative emotions,
each treatment protocol is a treatment process composed of a plurality of treatment methods,
the treatment protocol provision step provides the personalized treatment protocols for the patients by combining the treatment methods selected from a functional treatment method group, an emotional treatment method group, and a cognitive treatment method group on the basis of the classification results, and
the diagnostic step, the classification step, and the treatment protocol provision step are performed by using deep learning-based artificial intelligence algorithms.

10. The method of claim 9, wherein the treatment groups are composed of a functional treatment group, an emotional treatment group, and a cognitive treatment group.

11. The method of claim 8 or 9, wherein the scale for the tinnitus symptoms is derived by reflecting the tinnitus test results.

12. The method of claim 8 or 9, wherein the scale for the tinnitus-related negative thoughts and the scale for the tinnitus-related negative emotions are derived by reflecting the medical interview results.

13. The method of claim 8 or 9, wherein, as items that alleviate or directly treat the tinnitus symptoms, the functional treatment method group comprises sound therapy and tinnitus retraining therapy.

14. The method of claim 8 or 9, wherein the emotional treatment method group comprises meditation therapy, relaxation therapy, sleep hygiene therapy, mindfulness therapy, and acceptance and commitment therapy.

15. The method of claim 8 or 9, wherein the cognitive treatment method group comprises thought record therapy and educational therapy on cognitive factors.
